# EUROPEAN PATENT SPECIFICATION

(11) **EP 2 170 295 B1**
(45) Date of publication and mention of the grant of the patent: **22.04.2015**
(21) Application number: 08775312.5
(22) Date of filing: 23.07.2008
(51) Int. Cl.: A61K 9/20, A61K 31/4422

(54) **IMPROVED PHARMACEUTICAL COMPOSITION CONTAINING DIHYDROPYRIDINE CALCIUM CHANNEL ANTAGONIST AND METHOD FOR THE PREPARATION THEREOF**
VERBESSERTE PHARMAZEUTISCHE ZUSAMMENSETZUNG MIT DIHYDROPYRIDIN-CALCIUMKANAL-ANTAGONIST UND HERSTELLUNGSVERFAHREN DAFÜR
COMPOSITION PHARMACEUTIQUE AMÉLIORÉE CONTENANT UN ANTAGONISTE DE CANAL CALCIQUE DE DIHYDROPYRIDINE ET SON PROCÉDÉ DE PRÉPARATION

(30) Priority: 23.07.2007 WO PCT/EP2007/006517
(43) Date of publication of application: 07.04.2010
(73) Proprietor: Pharmathen S.A., Pallini Attikis 15351 (GR); Recordati Ireland Limited, Ringaskiddy County Cork (IE)
(72) Inventor: KARAVAS, Evangelos, GR-15351 Pallini Attikis (GR); KOUTRIS, Makis, GR-15351 Pallini Attikis (GR); SAMARA, Vicky, GR-15351 Pallini Attikis (GR); MATSINGOU, Christina, GR-15351 Pallini Attikis (GR); ILIOPOULOU, Athina, GR-15351 Pallini Attikis (GR)
(74) Representative: Pistolesi, Roberto
(86) International application number: PCT/EP2008/059645
(87) International publication number: WO 2009/013306

(56) References cited:
- WO-A-2004/066910
- WO-A-2005/053689
- KERC J ET AL: "USE OF HYDROPHILIC CARRIERS IN ENHANCEMENT OF FELODIPINE DISSOLUTION" ACTA PHARMACEUTICA JUGOSLAVICA, vol. 41, no. 3, 1991, pages 259-266, XP008089880 ISSN: 0001-6667

## Description

### TECHNICAL FIELD OF THE INVENTION

The present invention relates to improved dosage forms such as tablets and capsules and in particular to a formulation for oral administration with enhanced bioavailability comprising a therapeutically effective quantity of a dihydropyridine calcium channel antagonist, and more particularly Lercanidipine or salt, derivative and polymorph thereof and a method for the preparation thereof.

### BACKGROUND OF THE INVENTION

Dihydropyridine calcium channel antagonist compounds, such as amlopidipine, nifedipine, lacidipine and lercanidipine, are compounds known to be extremely useful among others in the treatment of hypertension and coronary disease.

Lercanidipine (methyl 1, 1-N-trimethyl-N-(3, 3-diphenylpropyl) amino ethyl 1, 4-dihydro-6-dimethyl (3-nitrophenyl) pyridine-3, 5-dicarboxylate) is a highly lipophilic dihydropyridine calcium antagonist with long duration of action and high vascular selectivity. It is normally used in a dose of 10mg to 20mg once daily (marketed in Italy as Zanedip®), the maximum dose being about 30mg daily. Lercanidipine is rapidly absorbed following oral administration and peak plasma levels occurring 1, 5-3 hours following dosing, but it undergoes extensive first pass metabolism.

Dihydropyridine calcium channel antagonists have low water solubility and that result to low bioavailability of the active ingredient.

Drugs with low solubility in water (by which is meant having a solubility of less than 0.1 percent by weight in water at 20 "C) cause additional formulation problems due to their poor rate and extent of dissolution in aqueous media, including gastrointestinal fluids, which results in low absorption into systemic circulation after oral ingestion.

In order to make a composition containing such a drug that will enable maximum absorption from the gastrointestinal tract, it is necessary to incorporate in the composition a feature that increases the solubility of the drug to enable it to dissolve in the gastrointestinal fluids.

Various methods are already known for the industrial preparation of oral dosage forms comprising a dihydropyridine calcium channel antagonist, and in particular Lercanidipine or a pharmaceutical acceptable salt thereof as an active ingredient due to its useful therapeutical properties. However, the prior art has encountered substantial difficulties in the production of the oral solid formulations of a desirable bioavailability due to the poor solubility of said active ingredient.

It is known that active compounds in amorphous form often have a higher bioavailability than corresponding crystalline active compounds. DE-A-3 024 858 discloses a dosage form comprising nicardipine, a sparingly soluble dihydropyridine, used in its amorphous form in order to increase dissolution and absorption. Amorphous active ingredients usually should be carefully formulated because they have a tendency to recrystallize, resulting to bioavailability that is not reproducible or decreases significantly after certain storage periods due to degradation products.

EP 0 385 582 discloses nifedipine composition having a particle size of less than 100 microns. Although the control of the dissolution of nifedipine is achieved by processing the material to a large specific surface area, the small crystals of the active ingredient have the tendency to agglomerate and reform to larger particle sizes.

EP 0 557 244 discloses compositions which contain nifedipine which has been micronized to small crystals to increase solubility, along with a hydrophilic gel-forming polymer to slow-down and control the rate of dissolution and absorption. However, the smallest size to which nifedipine can be micronized using conventional equipment is about 1 micron, and this particle size is still not small enough to enable full dissolution and absorption of the nifedipine.
Moreover, unless the crystal size is carefully controlled to be the same in every batch of tablets, release characteristics may vary from batch to batch.

GB 1 456 618 discloses improving the dissolution and absorption of nifedipine by preparation of a solid solution of nifedipine in polyethylene glycol in the presence of a surfactant agent. EP 0 448 091 discloses a dihydropyridine with a surfactant but large quantities of surfactants usually cause irritation to the stomach of the patients.

In addition, the use of surfactants, solubilizing agents and certain excipients which have a particular surface frequently leads to administration forms in which the products are undesirably large. To facilitate swallowing, such tablets or capsules are frequently converted into specific forms, such as, for example, ellipsoids or longitudinal shapes, but this also no longer gives satisfactory results in products weighing more than 400 mg. More frequent taking of smaller products is also not a satisfactory solution.

Although each of the above patent documents represents an attempt to overcome the instability problems associated with pharmaceuticals compositions comprising dihydropyridine calcium channel antagonists, there still exists a need for improving the bioavailability of such active ingredients and, in particular, for rendering their bioavaliability independent from fast/fed conditions.

WO 2005/053689 A discloses controlled release compositions comprising lercanidipine dissolved or dispersed in a solid vehicle at ambient temperature, thus forming a solid dispersion.

### SUMMARY OF THE INVENTION

It is, therefore, an object of the present invention to provide an improved solid dosage formulation for oral administration containing a dihydropyridine calcium channel antagonist, and in particular Lercanidipine or salt thereof as an active ingredient, which overcomes the deficiencies of the prior art, enhances the bioavailability of the active ingredient and render it independent from fast/fed conditions.

Another aspect of the present invention is to provide a solid dosage formulation for oral administration containing a dihydropyridine calcium channel antagonist, and in particular Lercanidipine or salt thereof as an active ingredient, which is bioavailable and effective with sufficient self-life, good pharmacotechnical properties enhancing patient compliance and reducing possible side effects.

Moreover, another aspect of the present invention is to provide a solid dosage formulation for oral administration containing a dihydropyridine calcium channel antagonist, and in particular Lercanidipine or salt thereof as an active ingredient, which can be prepared in dosage forms of different strength by proportionally adjusting the quantities of the excipients and the active ingredient, thereby providing a pharmacotechnical linearity, without affecting the dissolution profile and bioavailability of the active ingredient.

A further aspect of the present invention is to provide a method for the preparation of a stable solid dosage formulation for oral administration containing a dihydropyridine calcium channel antagonist, and in particular Lercanidipine or salt thereof as an active ingredient, thereby enhancing the bioavailability of the active ingredient, being stable over a long period of time and improving the pharmacotechnical characteristics of the composition.

In accordance with the above objects of the present invention, a pharmaceutical composition for oral administration in solid dosage form is provided comprising a dihydropyridine calcium channel antagonist, and in particular Lercanidipine or a pharmaceutical acceptable salt, derivative and polymorph thereof as an active ingredients, and an amount of colloidal silicon dioxide from 5% to 25% by weight such as Aerosil as an agent to enhance bioavailability, said composition being obtainable according to the process of claim 1.

In particular, a preferred object of the invention is represented by a pharmaceutical composition for oral administration comprising Lercanidipine or a pharmaceutical acceptable salt, derivative and polymorph thereof, from 7% to 20% by weight, of colloidal silicon dioxide.

As it will be apparent from the description and the examples, the compositions of the present invention provide a bioavailability of the active principle which is about 15-25% higher than that achievable with the compositions currently available on the market; consequently, in the specific case of Lercanidipine, it is possible to obtain a bioavailability which is equivalent to that of Zanedip® 10 mg and Zanedip® 20 mg with an amount of Lercanidipine HCl of about 8 mg and 16 mg, respectively. Furthermore, the composition of the present invention has reduced or eliminated the dependency of the bioavaliability from fast/fed conditions.

According to another embodiment of the present invention, a process for the preparation of solid dosage forms for oral administration such as tablets, capsules and sachets, containing a dihydropyridine calcium channel antagonist, and in particular Lercanidipine or a pharmaceutical acceptable salt, derivative and polymorph thereof as an active ingredient is provided, which comprises:
- dissolving the total quantity of said active ingredient, a portion of a total quantity of colloidal silicon dioxide to enhance bioavailability and optionally a binder into a water/EtOH solvent;
- adding to the formed solution the remaining portion of colloidal silicon dioxide and an optional excipient such as a diluent, a binder, a disintegrant, a glidant, a lubricant and wet granulating;
- dissolving a wetting agent into a small quantity of water/EtOH solvent and kneading with the first solution;
- drying the wetted mass;
- sieving the dried mass and adding to the sieved mixture the total quantities of at least one optional excipient such as a binder, a wetting agent, a diluent, a disintegrant, a lubricant and/or a glidant and mixing until uniform, and
- formulating the resulting mixture in a solid dosage form either by compressing it into a desired tablet form or by filling capsules or sachets.

Further preferred embodiments of the present invention are defined in dependent claims 3 to 17.

Other objects and advantages of the present invention will become apparent to those skilled in the art in view of the following detailed description.

### BRIEF DESCRIPTION OF THE DRAWINGS

Figs. 1 and 2 show average plasma values for the composition of Example 1 according to the present invention.
Figs. 3 and 4 show average plasma values for the composition of Example 3 according to the present invention.
Figs. 5, 6, and 7 show X-RD spectrums of amorphous Lercanidipine HCl, placebo of composition of Example 3, composition of Example 3 according to the present invention.
Fig. 8 shows SEM of amorphous Lercanidipine HCl.
Fig. 9 shows SEM of fine dispersion of amorphous Lercanidipine HCl and colloidal silicon dioxide according to the present invention.
Fig. 10 shows the dissolution profile of the composition of Example 4 (containing 16 mg of Lercanidipine HCl) compared to Zanedip® (containing 20 mg of Lercanidipine HCl).
Fig. 11 shows the dissolution profile of the composition of Example 5 (containing 8 mg of Lercanidipine HCl) compared to Zanedip® (containing 10 mg of Lercanidipine HCl).
Figs. 12 and 13 shows the XRD spectra of the compositions of Example 4 and 5, respectively.
Figs 14 and 15 shows the dissolution profiles under simulated fast/fed conditions of the composition of Example 4 and of Zanedi® 20 mg, respectively.

### DETAILED DESCRIPTION OF THE INVENTION

For the purposes of the present invention, a pharmaceutical composition comprising a poorly soluble active ingredient (Dihydropyridine calcium channel antagonist e.g. Lercanidipine or pharmaceutical acceptable salt thereof) is considered to be "stable" if said ingredient degradates less or more slowly than it does on its own and/or in known pharmaceutical compositions. Furthermore, it is considered to render the bioavailability of the active ingredient independent from fast/fed conditions when it provides essentially the same bioavailability of the active ingredient independently from whether it is administered to a patient who is in a fast or a fed condition.

The active ingredient (a dihydropyridine calcium channel antagonist, and in particular Lercanidipine or a pharmaceutical acceptable salt thereof) contained in a dosage form is "enhanced bioavailable", if when administered in a dosage form is released from the dosage form, absorbed and subsequently reaches higher concentration levels in plasma than the marketed products containing the same quantity of the same active ingredient and intended for the same use.

Although the pharmaceutical composition may be in various forms, the preferred solid forms are tablets, capsules and caplets.

It has been surprisingly found that the object of the present invention is achieved by employing colloidal silicon dioxide such as Aerosil™ in order to enhance the bioavailability of the active ingredient.

As already mentioned dihydropyridine calcium channel antagonists such as Lercanidipine HCl have very poor solubility thus reflecting in poor bioavailability of the active substance.

Colloidal silicon dioxide is a submicroscopic fumed silica with a particle size of about 15nm. It is a light, loose, bluish-white-colored, odourless, tasteless, nongritty amorphous powder. Colloidal silicon dioxide is widely used in pharmaceuticals. Its small particle size and large specific surface area give it desirable flow characteristics that are exploited to improve the flow properties of dry powders

When colloidal silicon dioxide is incorporated in a pharmaceutical composition according to the present invention a fine dispersion of amorphous particles of the active ingredient on the surface of colloidal silicon dioxide is being formed resulting in a one-phase system. Said one-phase system improves the solubility of the active ingredient.

The active ingredient (dihydropyridine calcium channel antagonists such as Lercanidipine or salt, derivative and polymorph thereof) and a suitable amount of colloidal silicon dioxide such as Aerosil™ are being dissolved in a solvent in order to form a fine dispersion, and subsequently a binder is being admixed. The remaining portion of the colloidal silicon dioxide and an optional excipient is being added in the solution and wet granulating. After drying the wetted mass and sieving the dried mass, any optional additional excipient is then added. The composition is then mixed until uniform. The resulting composition may then be compressed.

Moreover, any excipient may optionally be added to the above composition, provided that they are compatible with the active ingredient of the composition, in order to overcome problems associated with the poor flow properties and unfavorable phannacotechnical characteristics of these substances, and in order to increase the stability of the drug and the self-life of the pharmaceutical product, and provide a product exhibiting excellent bioavailability.

The present invention can be applied in the formulation of tablets, capsules, caplets, sachets or other solid dosage forms for oral or sub-lingual administration of an active ingredient having solubility and bioavailability problems.

Furthermore, it is possible to prepare dosage forms of different strength using appropriate quantity of the same composition, thereby limiting the cost of production and minimizing the number, and consequently the cost, of clinical studies required for the approval of the product by the authorities.
The manufacturing process for preparation according to the present invention is simpler and inexpensive in comparison to any other conventional method.

Therefore, in a first embodiment, the present invention provides a pharmaceutical composition comprising from about 0.5% to 30% by weight of Lercanidipine or salt thereof and from about 3% to 30% by weight of Colloidal silicon dioxide, said composition being obtainable according to the process of claim 1. The weight ratio of Lercanidipine or salt thereof to Colloidal silicon dioxide is preferably 10:1 to 1:60.

More preferred pharmaceutical compositions according to the present invention comprise approximately 3% to 30%, more preferably 5% to 25% and most preferably 7% to 20% by weight of Colloidal silicon dioxide, such as Aerosil™. Such preferred compositions comprise from 3% to 25% by weight of Lercanidipine or a pharmaceutical acceptable salt, derivative and polymorph thereof, preferably from 5% to 10% by weight, more preferably about 8% by weight.

A binder, if present, may be generally present in an amount of from 5% to 20% by weight, preferably in amounts of up to about 15 % by weight; the wetting agent, if present, may generally be present in an amount of up to about 5 % by weight, preferably of about 2.5% by weight.

The pharmaceutical compositions of the invention normally contain a diluent, which may be present in amounts of from 40% to 65% by weight, preferably of from 45% to 60% by weight. Such compositions may also contain a disintegrant, which is preferably present in amounts of from 5% to 15% by weight, more preferably of about 10% by weight.

According to another embodiment, the pharmaceutical compositions of the present invention comprise an internal phase and an external phase; according to a preferred embodiment, said external phase comprises, and preferably consists of, magnesium stearate.

The preferred pharmaceutical compositions are in the form of solid dosage forms for oral or sub-lingual administration such as tablets, capsules, caplets, troches, pastilles, pills, lozenges and the like, in all shapes and sizes, coated or uncoated.

All percentages stated herein are weight percentages based on total composition weight, unless otherwise stated.

Another embodiment of the present invention is the use of the wet granulation process for the preparation of solid dosage forms for oral administration such as tablets, capsules and sachets containing Lercanidipine or salt, derivative and polymorph thereof. Said wet granulation process comprises:
- dissolving the total quantity of a dihydropyridine calcium channel antagonist, and in particular Lercanidipine or a pharmaceutical acceptable salt thereof as an active ingredient, a portion of a total quantity of colloidal silicon dioxide (preferably from 40 to 60% of the total weight) to enhance bioavailability and optionally a binder into a water/EtOH solvent;
- adding to the formed solution the remaining portion of colloidal silicon dioxide and an optional excipient such as a diluent, a binder, a disintegrant, a glidant, a lubricant and wet granulating;
- dissolving a wetting agent into a small quantity of water/EtOH solvent and kneading with the first solution;
- drying the wetted mass;
- sieving the dried mass and adding to the sieved mixture the total quantities of at least one optional excipient such as a binder, a wetting agent, a diluent, a disintegrant, a lubricant and/or a glidant and mixing until uniform, and
- formulating the resulting mixture in a solid dosage form either by compressing it into a desired tablet form or by filling capsules or sachets.

The pharmaceutical compositions of the present invention may also contain one or more additional formulation ingredients selected from a wide variety of excipients. According to the desired properties of the composition, any number of ingredients may be selected, alone or in combination, based upon their known uses in preparation of solid dosage form compositions.

Such ingredients include, but are not limited to, diluents, binders, compression aids, disintegrants, surfactants, wetting agents, antioxidants, glidants, lubricants, flavors, water scavengers, colorants, sweetener, coating agents and preservatives.

The optional excipients must be compatible with the dihydropyridine calcium channel antagonist or the salt thereof so that it does not interfere with it in the composition.
Diluents may be, for example, calcium carbonate, calcium phosphate dibasic, calcium phosphate tribasic, calcium sulfate, microcrystalline cellulose, microcrystalline silicified cellulose, powdered cellulose, dextrates, dextrose, fructose, lactitol, lactose anhydrous, lactose monohydrate, lactose dihydrate, lactose trihydrate, mannitol sorbitol, starch, pregelatinized starch, sucrose, talc, xylitol, maltose maltodextrin, maltitol.

Binders may be, for example, acacia mucilage, alginic acid, carbomer, carboxymethylcellulose calcium, carboxymethylcellulose sodium, microcrystalline cellulose, powdered cellulose, ethyl cellulose, gelatin, liquid glucose, guar gum, hydroxyethyl cellulose, hydroxypropyl cellulose, hydroxypropylmethyl cellulose, maltodextrin, methylcellulose, polydextrose, polyethylene oxide, povidone, sodium alginate, starch paste, pregelatinized starch, sucrose.
Disintegrants may be, for example, alginic acid, carbon dioxide, carboxymethylcellulose calcium, carboxymethylcellulose sodium, microcrystalline cellulose, powdered cellulose, croscarmelose sodium, crospovidone, sodium docusate, guar gum, hydroxypropyl cellulose, methylcellulose, polacrilin potassium, poloxamer, povidone, sodium alginate, sodium glycine carbonate, sodium laulyl sulfate, sodium starch glycolate, starch, pregelatinized starch.
Wetting agents may be, polyoxyethylene-polyoxypropylene co-polymers and block co-polymers, commercially available as Pluronic™ or Poloxamer™, ethoxylated cholesterins, commercially available as Solulan™ vitamin derivatives, e. g. vitamin E derivatives such as tocopherol polyethylene glycol succinate (TPGS), sodium dodecylsulfate or sodium laurylsulfate; a bile acid or salt thereof, for example cholic acid, glycolic acid or a salt.
Glidants may be, for example, calcium silicate, powdered cellulose, starch, talc, colloidal silicon dioxide.
Lubricants may be e.g. polyethylene glycol 4000, polyethylene glycol 6000, sodium lauryl sulfate, starch, talc, magnesium stearate, glyceryl behenate, hydrogenated castor oil, stearic acid, glyceryl palmitostearate, glyceryl monostearate, sodium stearyl fumarate.

According to another embodiment, the pharmaceutical composition of the present invention is in solid dosage form and each form contains from 7 to 9 mg or from 14 to 18 mg of Lercanidipine hydrochloride, preferably about 8 mg or about 16 mg of Lercanidipine hydrochloride. In particular, according to particularly preferred embodiments, each form may contain:
- about 16 mg of Lercanidipine hydrochloride, about 80 mg of lactose monohydrate, about 16 mg of microcrystalline cellulose, about 20 mg of sodium starch glycolate, about 30 mg ofpolyvinylpyrrolidone, about 31 mg of colloidal silicon dioxide, about 5 mg of polyoxyethylene-polyoxypropylene copolymer, about 2 mg of magnesium stearate; or
- about 8 mg of Lercanidipine hydrochloride, about 45 mg of lactose monohydrate, about 8 mg of microcrystalline cellulose, about 10 mg of sodium starch glycolate, about 10 mg of polyvinylpyrrolidone, about 15.50 mg of colloidal silicon dioxide, about 2.50 mg of polyoxyethylene-polyoxypropylene copolymer, about 1 mg of magnesium stearate.

The following examples illustrate preferred embodiments in accordance with the present invention without limiting the scope of the invention:

### EXAMPLES

### Example 1 : Tablet of 20 mg Lercanidipine (Composition 1) (not according to the invention)

| **Ingredients** | **%** | **20mg Tablet** |
|---|---|---|
| **Internal Phase** | | |
| Lercanidipine HCl | 10.00 | 20.00 |
| Microcellac | 40.00 | 80.00 |
| Microcrystalline Cellulose | 25.00 | 50.00 |
| Starch 1500 | 17.00 | 34.00 |
| Primojel | 2.00 | 4.00 |
| HPC | 5.00 | 10.00 |
| *Purified Water* | | *30.00* |
| *EtOH* | | *24.00* |

| **External phase** | | |
|---|---|---|
| Mg Stearate | 1.00 | 2.00 |
| | **Total weight** | 200.00 |

Tablets of the above formulation were prepared according to the following manufacturing process: HPC was dissolved in a water/EtOH solvent. Lercanidipine HCl was mixed with Microcellac, Microcrystalline Cellulose, Starch 1500 and Primojel to form a homogenous mixture. The above mixture was kneaded with the solution of HPC. The granular mass was dried. Finally Mg Stearate was added to the dried granule and mixed until complete homogeneity. The resulting granule was compressed into tablets.

The produced tablets were tested for hardness, friability, disintegration, and water content. All tests were performed according to European Pharmacopoeia 5.1 and were well within the specifications.

### Example 2 : Tablet of 20 mg Lercanidipine (Composition 2) (not according to the invention)

| **Ingredients** | **%** | **20mg Tablet** |
|---|---|---|
| **Internal Phase** | | |
| Lercanidipine HCl | 10.00 | 20.00 |
| Lactose Monohydrate | 35.00 | 70.00 |
| Microcrystalline Cellulose | 22.50 | 45.00 |
| Starch 1500 | 15.00 | 30.00 |
| Primojel | 8.00 | 16.00 |
| Tween 20 | 1.00 | 2.00 |
| *Purified Water* | | *45.00* |
| *EtOH* | | *19.80* |

| **External phase** | | |
|---|---|---|
| Microcrystalline Cellulose | 7.50 | 15.00 |
| Mg Stearate | 1.00 | 2.00 |
| | **Total weight** | 200.00 |

Tablets of the above formulation were prepared according to the following manufacturing process: Tween 20 was dissolved in 20mg of water (solution 1). Lactose monohydrate, Lercanidipine HCl and half of Primojel's quantity were dissolved in the remaining quantity of water and EtOH and mixed (solution 2). Solution 1 and 2 were combined and subsequently a mixture of microcrystalline cellulose, Starch 1500 and the remaining quantity of Primojel were added and mixed. The granular mass was dried. Microcrystalline cellulose was added to the dried granule and mixed. Finally, Mg Stearate was added to the granule and mixed until complete homogeneity. The resulting granule was compressed into tablets.

### Example 3: Tablet of 20 mg Lercanidipine (Composition 3)

| **Ingredients** | **%** | **20mg Tablet** |
|---|---|---|
| **Internal Phase** | | |
| Lercanidipine HCl | 10.00 | 20.00 |
| Lactose Monohydrate | 48.00 | 96.00 |
| Microcrystalline Cellulose | 8.00 | 16.00 |
| Primojel | 10.00 | 20.00 |
| PVP | 5.00 | 10.00 |
| Aerosil | 15.50 | 31.00 |
| Poloxamer | 2.50 | 5.00 |
| *Purified Water* | | *43.30* |
| *EtOH* | | *34.70* |

| **External phase** | | |
|---|---|---|
| Mg Stearate | 1.00 | 2.00 |
| | **Total weight** | 200.00 |

Tablets of the above formulation were prepared according to the following manufacturing process: Lercanidipine HCl and half of the quantity of Aerosil™ were dissolved/dispersed in a water/EtOH solvent, and subsequently PVP was added. Lactose monohydrate and the remaining portion of the quantity of Aerosil™ were admixed, added to the previous solution and kneaded. Then Primojel was added to the above solution. Poloxamer was dissolved in a small quantity of water/EtOH solvent and kneaded with the previous solution. The granular mass was dried and sieved. Finally, Mg Stearate was added to the dried granule and mixed to complete homogeneity. The resulting granule was compressed into tablets.

### Example 4: Tablet of 16 mg Lercanidipine (Composition 4)

| **Ingredients** | **%** | **mg Tablet** |
|---|---|---|
| **Internal Phase** | | |
| Lercanidipine HCl | 8.00 | 16.00 |
| Lactose Monohydrate | 40.00 | 80.00 |
| Microcrystalline Cellulose | 8.00 | 16.00 |
| Sodium starch glycolate (Primojel) | 10.00 | 20.00 |
| Polyvinylpyrrolidone (povidone/kollidon K30) | 15.00 | 30.00 |
| Colloidal silicon dioxide (Aerosil) | 15.50 | 31.00 |
| Polyoxyethylene-polyoxypropylene copolymer (Poloxamer 407) | 2.50 | 5.00 |
| *Purified Water* | | *94.50* |
| *EtOH* | | *40.00* |

| **External phase** | | |
|---|---|---|
| Mg Stearate | 1.00 | 2.00 |
| **Total** | **100.00** | **200.00** |

### Example 5: Tablet of 8 mg Lercanidipine (Composition 5)

| **Ingredients** | **%** | **mg Tablet** |
|---|---|---|
| **Internal Phase** | | |
| Lercanidipine HCl | 8.00 | 8.00 |
| Lactose Monohydrate | 45.00 | 45.00 |
| Microcrystalline Cellulose | 8.00 | 8.00 |
| Sodium starch glycolate (Primojel) | 10.00 | 10.00 |
| Polyvinylpyrrolidone (povidone/kollidon K30) | 10.00 | 10.00 |
| Colloidal silicon dioxide (Aerosil) | 15.50 | 15.50 |
| Polyoxyethylene-polyoxypropylene copolymer (Poloxamer 407) | 2.50 | 2.50 |
| *Purified Water* | | *47*.*25* |
| *EtOH* | | *20*.*00* |

| **External phase** | | |
|---|---|---|
| Mg Stearate | 1.00 | 1.00 |
| **Total** | **100.00** | **100.00** |

The tablets of the compositions of examples 4 and 5 have been manufactured by using the same process of example 3. They essentially differ therefrom because Lercanidipine HCl is in crystalline form (rather than amorphous) and it is present in a lower amount (i.e. 8% by waight rather than 10%); furthermore, they also have a lower content of lactose monohydrate and a higher content of PVP.

### Comparative studies

One of the most critical pharmacotechnical tests is the dissolution test as it is strongly correlated with the bioavailability of the product. For the dissolution method a Paddles Apparatus II was run at 75rpm, 37°C±0.5°C, for 30min, while as dissolution medium a buffer pH=1,2 was used.

Dissolution rate results for each composition tested are given in Table 1. The results show that all three compositions are not completely dissolved in about 30 minutes.

**TABLE 1: Dissolution profiles of the compositions of Examples 1, 2 and 3**

| **Time (min)** | **Composition 1** | **Composition 2** | **Composition 3** |
|---|---|---|---|
| **5** | 12,25 | 49,31 | 19,96 |
| **10** | 25,99 | 60,18 | 31,33 |
| **15** | 35,94 | 65,80 | 69,07 |
| **20** | 42,08 | 70,11 | 80,69 |
| **25** | 47,91 | 73,29 | 88,01 |
| **30** | 55,29 | 74,83 | 90,73 |

It is a generally known problem for pharmaceutical compositions of low solubility active ingredients that even though dissolution test provides satisfactory results, many times in vivo results depart from what it is expected. For this kind of drugs with low absorption (lower than 10 %) due to low solubility and high first pass metabolism the dissolution test is not so discriminative, thus only the pharmacokinetic study results are representative regarding the formulations.

Another objects of the present invention was to prepare a pharmaceutical composition that is stable, said active ingredient does not degradate and remains in amorphous form for a long period of storage time. Therefore, prior to the clinical trials, the three compositions were packed in PVC/PE/PVDC Aluminum blisters and exposed to normal (25°C±2°C/60%±5% RH), and accelerated (40°C±C2°C/75%±5% RH) stability studies according to the current ICH guidelines. The stability results after six months are shown in the table below.
The results show that Lercanidipine is more stable when colloidal silicon dioxide is incorporated in the formulation.
The specific tests and results are described in the stability table (TABLE 2).

**TABLE 2: Stability results for compositions 1, 2 and 3 directly after preparation and after 6 months of storage in normal and accelerated conditions.**

| **0 MONTHS** | | | |
|---|---|---|---|
| **IMPURITIES** | **Comp 1** | **Comp 2** | **Comp 3** |
| Imp A NMT 0.15% | ND | ND | ND |
| Imp B NMT 0.15% | ND | ND | ND |
| Imp C NMT 0.15% | 0,04% | 0,05% | 0,03% |
| Imp D NMT 0.50% | 0,04% | 0,04% | 0,04% |
| Unknown NMT 0.20% | ND | ND | ND |
| Total NMT 1.2% | 0,08% | 0,09% | 0,07% |

| **6 MONTHS** | | | |
|---|---|---|---|
| **IMPURITIES** | **Comp 1** | **Comp 2** | **Comp 3** |
| **25°C±2°C**/**60**%**±5**% **RH** | | | |
| Imp A NMT 0.15% | ND | ND | ND |
| Imp B NMT 0.15% | ND | ND | ND |
| Imp C NMT 0.15% | 0,04% | 0,06% | 0,03% |
| Imp D NMT 0.50% | 0,04% | 0,04% | 0,04% |
| Unknown NMT 0.20% | ND | ND | ND |
| Total NMT 1.2% | 0,08% | 0,10% | 0,07% |
| | | | |

| **40°C±2°C/75%±5% RH** | | | |
|---|---|---|---|
| Imp A NMT 0.15% | ND | ND | ND |
| Imp B NMT 0.15% | ND | 0,01% | ND |
| Imp C NMT 0.15% | 0,06% | 0,06% | 0,05% |
| Imp D NMT 0.50% | 0,18% | 0,17% | 0,16% |
| Unknown NMT 0.20% | ND | ND | ND |
| Total NMT 1.2% | 0,24% | 0,24% | 0,21% |

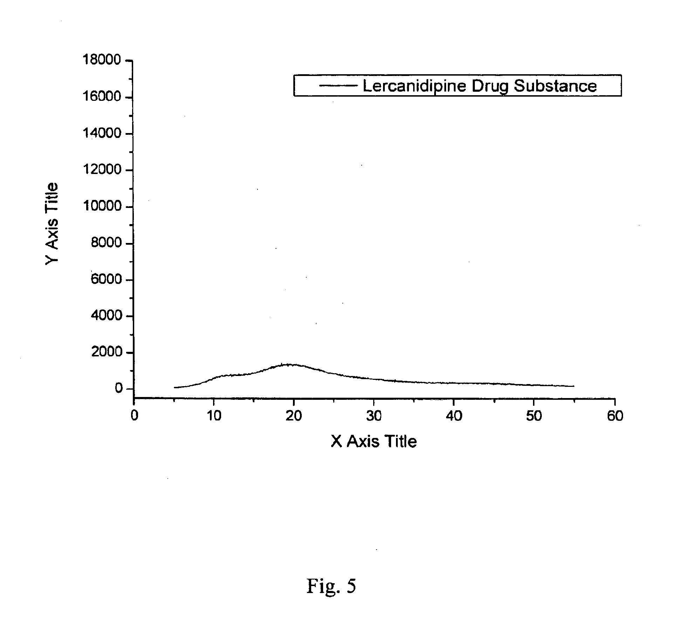

According to another aspect of the present invention, the active substance should remain in amorphous state after compression and should not convert in crystalline form.
As shown in Fig. 5 by the X-RD analysis Lercanidipine is completely amorphous since only a broad peak is recorded with a maximum at around 2θ = 20 deg. The crystal properties remain also unchanged after six months in the same conditions when the mixture is incorporated in a pharmaceutical composition with other excipients. No peaks corresponding to any crystalline form of Lercanidipine are observed directly after preparation or after 6 months storage indicating that the mixture is stabilized.

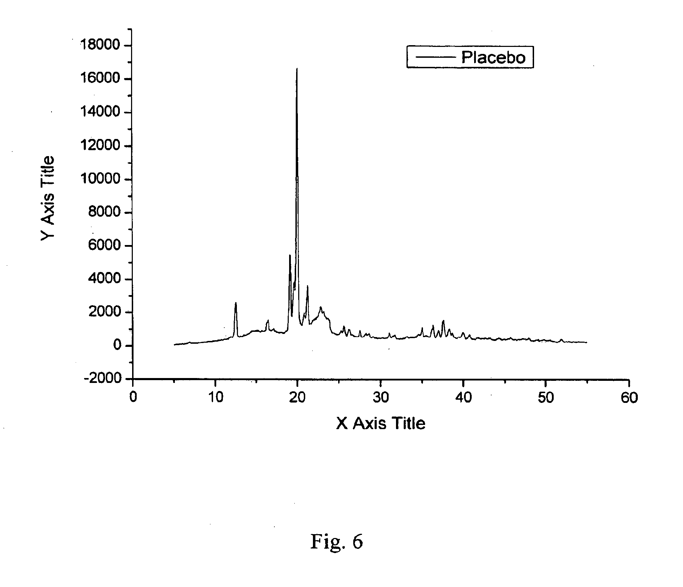

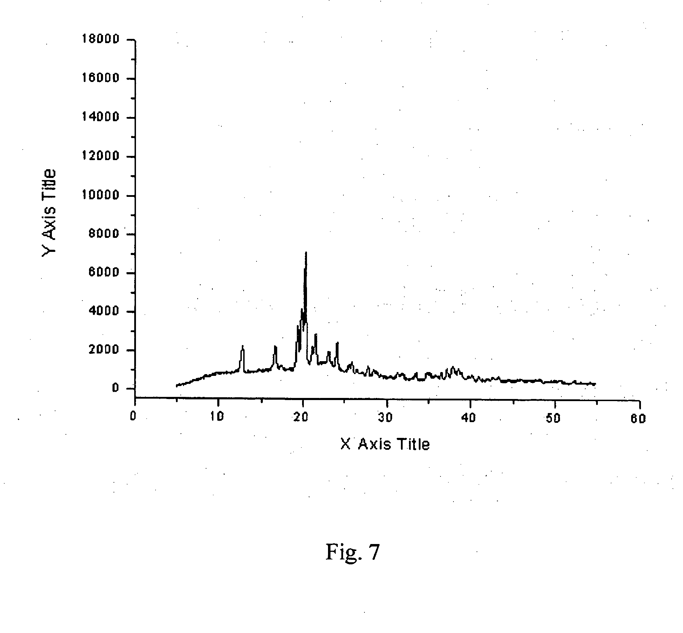

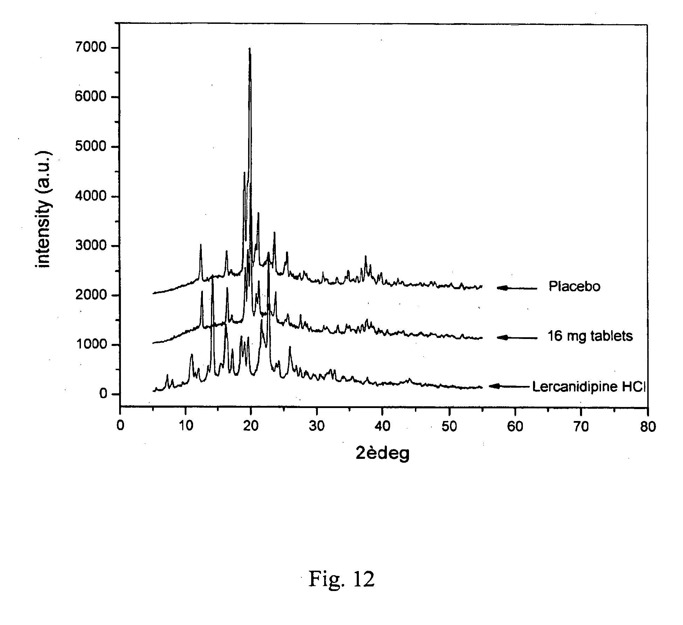

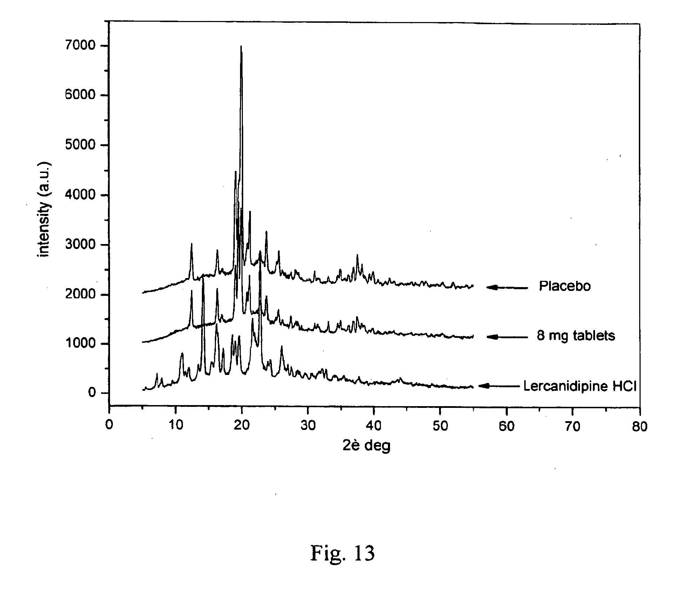

Tablets of the composition of Example 3 have the main peaks obtained at approximately 28=12.7, 16.6, 19.2, 19.7, 20.2, 21.4, 23.0, 36.4, 37.8 degrees which are also found in the placebo tablets (Fig. 6 and 7). The XRD of compositions 4 and 5 are reported in figures 12 and 13, respectively.
The bioavailability and pharmacokinetic profile of all five compositions of the present invention were determined in "in vivo" single-dose studies.

A single-dose study was conducted in 12 healthy volunteers using a formulation prepared with amorphous Lercanidipine HCL according to Examples 1, 2 and 3.

The reference compound was a 20mg Lercanidipine HCL tablet (Carmen 20 mg) that consists of the active ingredient, lactose, microcrystalline cellulose, sodium starch glycolate, povidone, magnesium stearate and opadry pink (Composition B); that is, a tablet having the same composition of Zanedip® 20 mg.

Each patient received a single oral 20mg dose of composition 1 of Example 1 and a tablet of Composition B equal to 20 mg of active ingredient, at different times. Blood samples were taken at different times and the plasma concentrations of Lercanidipine were determined.

In the pharmacokinetic analysis of composition 1 according to Example 1, R-Lercanidipine and S-Lercanidipine are measured separately (chiral method). Table 3 shows the main pharmacokinetic parameters obtained from the test.

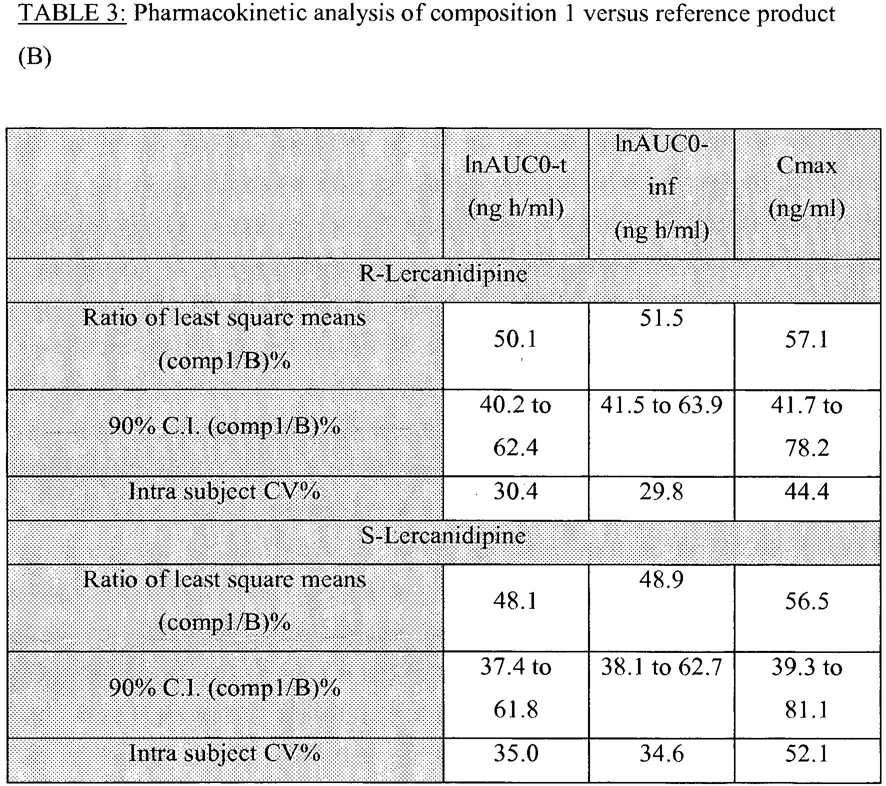

wherein:
C max = (peak concentration) is the highest concentration reached by the drug in plasma after dosing;
AUC₀₋ₜ =(area under the curve) is the total area under the time - plasma concentration curve, from time 0 to the last measurable concentration, as calculated by the linear trapezoidal method; it represents a measure of the bioavailability of the drug.
AUC_{0-inf} =(area under the curve) is the total area under the time- plasma concentration curve from time 0 to infinity. AUCinf is calculated as the sum of AUC 0-t plus the ratio of the last measurable plasma concentration to the elimination rate constant.
These data show that the properties of the two formulations are comparable with respect to the main pharmacokinetic parameters.

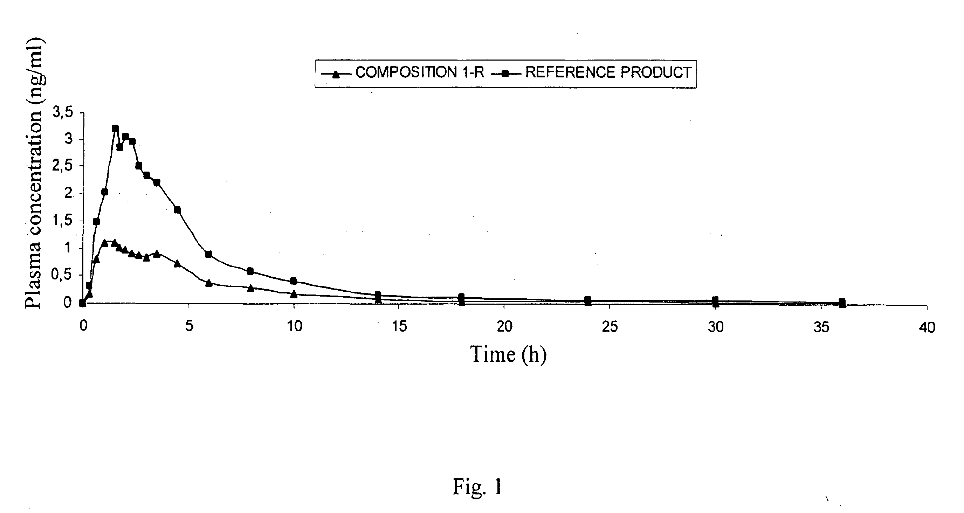

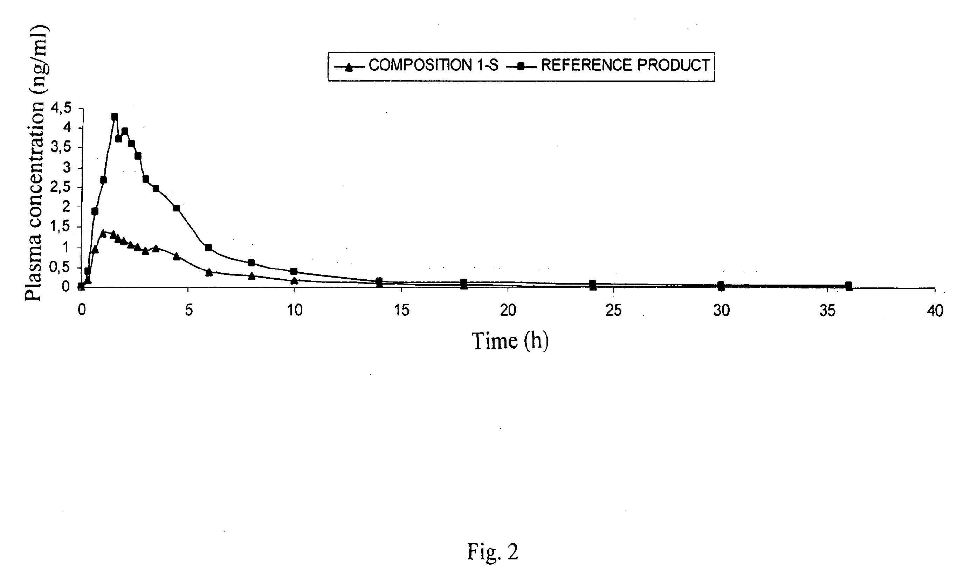

Consequently, it has been found that composition 1 (20 mg tablet) has a relative bioavailability of approximately 50% compared to the marketed Lercanidipine HCl 20 mg tablet (Fig. 1 and 2).

Moreover, a single-dose study was conducted in 12 human volunteers in a randomised two-way crossover study, comparing the dosage form of composition of Example 2 with the dosage form of composition B. Plasma samples were removed and tested for Lercanidipine at intervals.
In the pharmacokinetic analysis of the composition 2 according to Example 2, the racemic mixture of Lercanidipine is measured. Table 4 shows the main pharmacokinetic parameters obtained from the test.

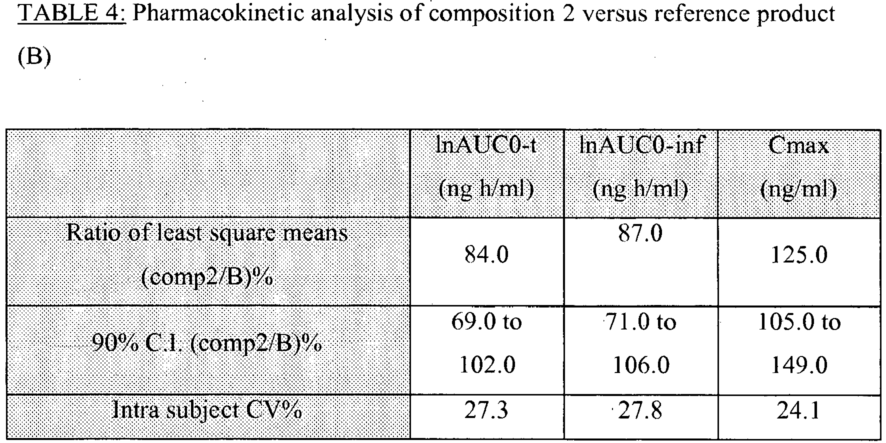

Consequently, it has been found that composition 2 (20 mg tablet) has a relative bioavailability of 84% compared to the marketed Lercanidipine HCl 20 mg tablet.

Further, a single-dose study was conducted in 72 human volunteers in a randomised two-way crossover study, comparing the dosage form of composition of Example 3 with the dosage form of composition B. Plasma samples were removed and tested for Lercanidipine at intervals.

In the pharmacokinetic analysis of the composition 3 according to Example 3, R-Lercanidipine and S-Lercanidipine are measured separately (chiral method). Table 5 shows the main pharmacokinetic parameters obtained from the test.

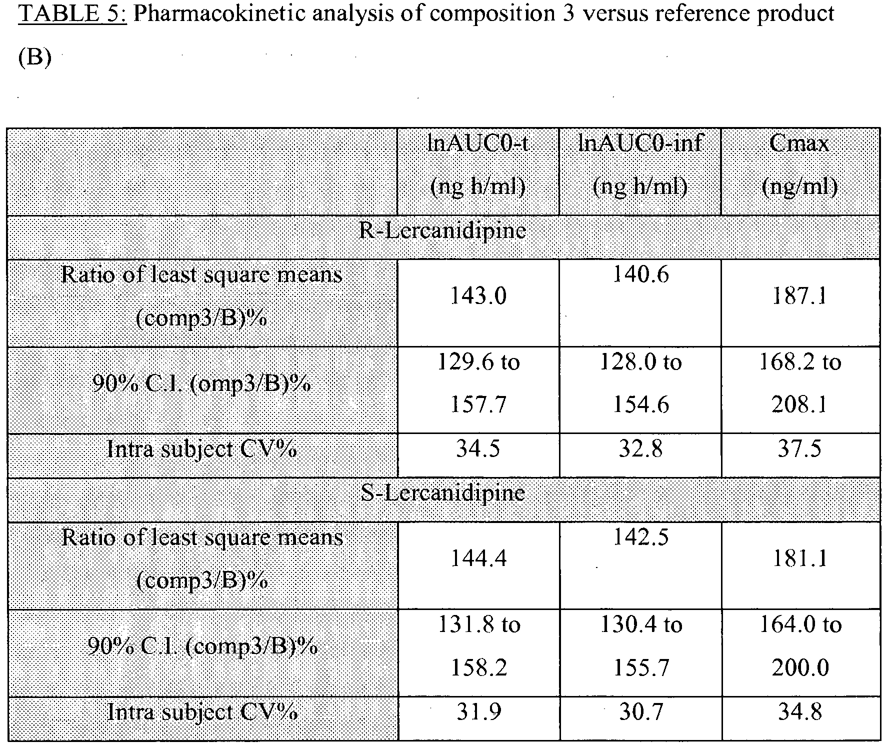

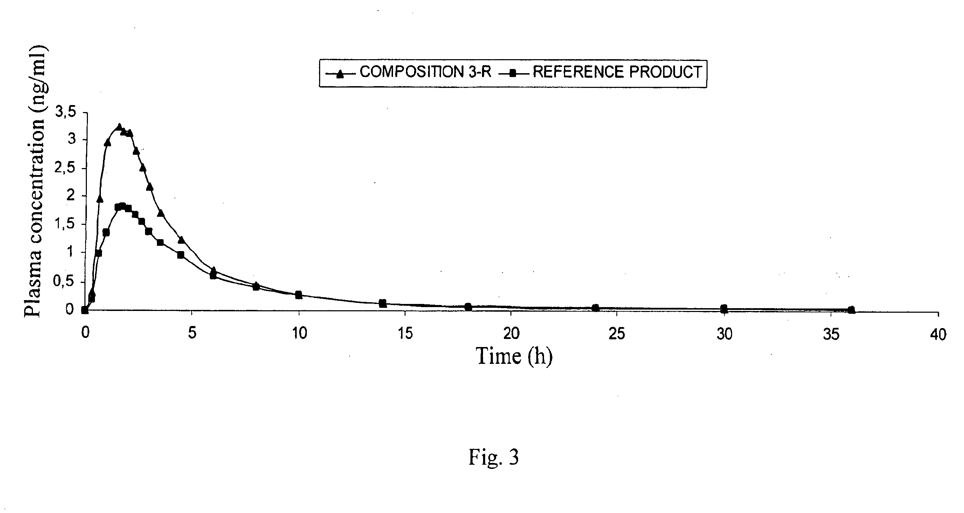

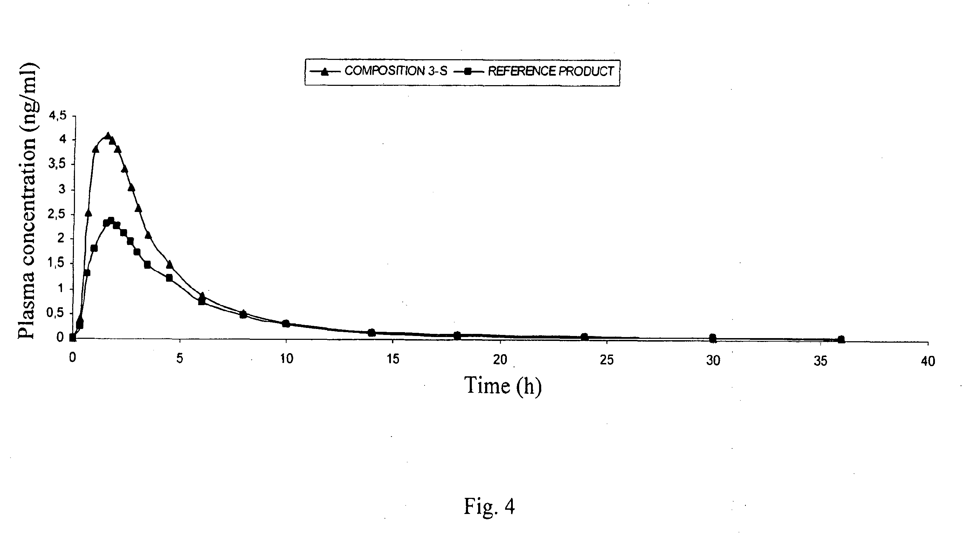

Consequently, it has been found that composition 3 (20 mg tablet) has a relative bioavailability of approximately 144% compared to the marketed Lercanidipine HCl 20 mg tablet (Fig 3 and 4).
The in vivo results indicate that concentration level of the active substance in plasma for composition 1 is approximately 50% of the level of the reference product. Composition 2 showed 68% increase in plasma concentration in comparison with composition 1. Surprisingly, composition 3 that is at approximately 144% of the level of the reference product has an increase of 71% and 188% in comparison with compositions 2 and 1, respectively.
The composition 3 of the present invention can therefore be considered, with respect the pharmacological performance, the best bioavailable solid formulations currently available.

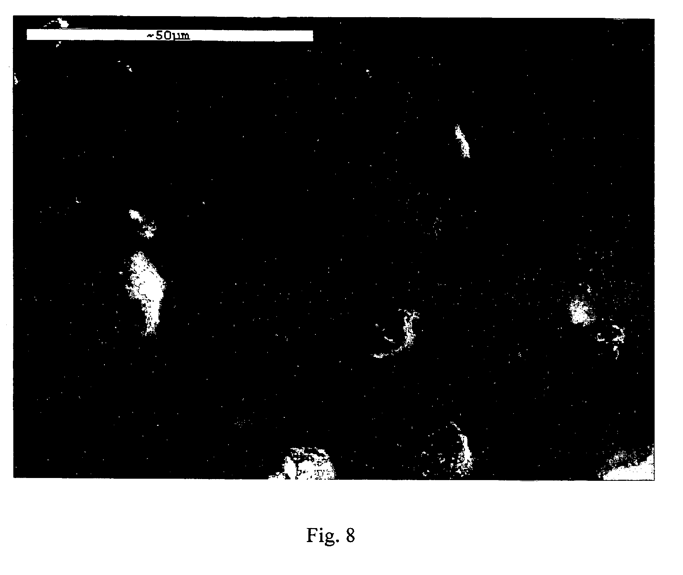

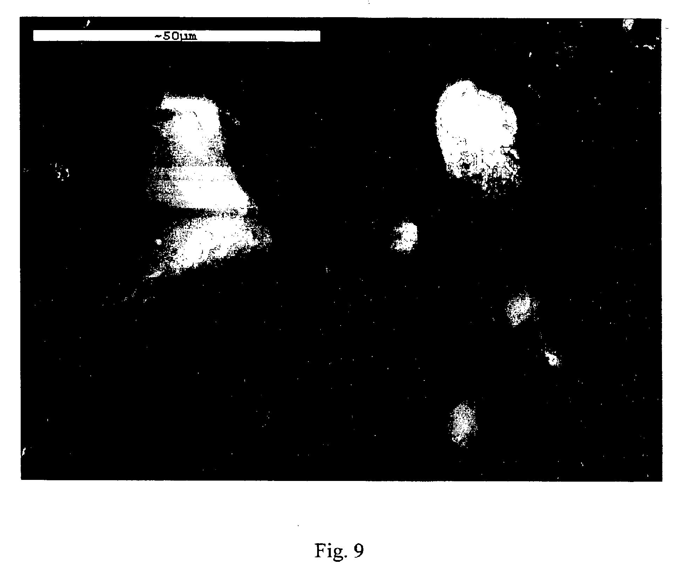

The increased bioavailability of composition 3 can be attributed to the formation of a fine dispersion between colloidal silica dioxide and Lercanidipine HCl. The optimal dispersion was verified by scanning electron microscopy (SEM) analysis and as it can be seen from Fig. 8 and 9 no particles or agglomerates of the drug substance were observed.
The very large surface of Aerosil™, on which the active ingredient is absorbed, results to an increase of the specific surface area and contribute to the excess of bioavailability.
The presence of the binder facilitates the homogenous distribution of the active ingredient on the surface of the Aerosil™ particles.

A further comparative study was carried out in order to compare the food effect of the compositions of examples 4 and 5 with respect to Zanedip® 20 mg and 10 mg, respectively.
More in details, the composition of example 4 has been administered to 12 healthy people with 240 mL of water, 15 minutes before administration of a standard breakfast or under fasting conditions; the same has been done with the composition of example 5. Zanedip® 20 mg has been administered to the same number/type of people with 240 mL of water, 15 minutes before administration of a standard breakfast; the same has been done with Zanedip® 10 mg. The effect of food on Zanedip® under these fed conditions has been calculated with an *indirect method.*
In all cases the breakfast consisted of: 1 buttered English muffin, 1 fried egg, 1 slide of American cheese, 1 slice of Canadian bacon, 1 serving of hash brown potatoes, 240 mL of whole milk, 180 mL of orange juice. The sampling schedule was: 0.333, 0.75, 1, 1.333, 1.667, 2, 2.333, 2.667, 3, 3.5, 4.5, 6, 8, 10, 14, 18, 24, 30 and 36 hours. The results are reported below.
i). The effect of food on the 16 mg formulation of example 4 is:
   AUCfed/AUCfast=103,6 %
   Cmax fed/Cmax fast=106,9%
ii). The effect of food on the 8 mg formulation of example 5 is:
   AUCfed/AUCfast=125,8 %
   Cmax fed/Cmax fast=118,0 %
iii). The effect of food on Zanedip® 20 mg is:
   AUCfed/AUCfast=125,2 %
   Cmax fed/Cmax fast=160,3 %
iv). The effect of food on Zanedip® 10 mg is:
   AUCfed/AUCfast=163,4 %
   Cmax fed/Cmax fast=178,5 %

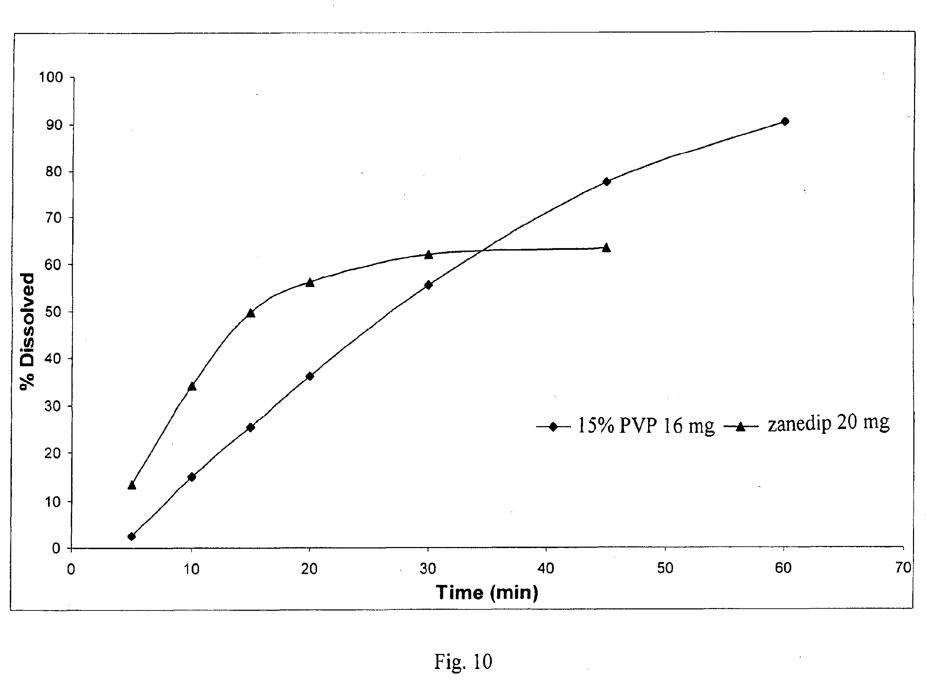

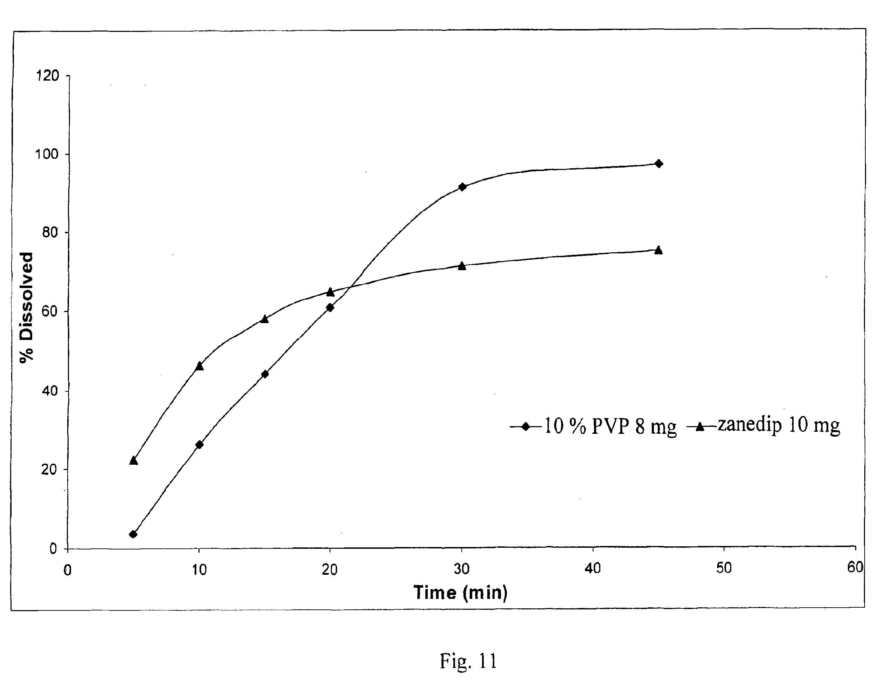

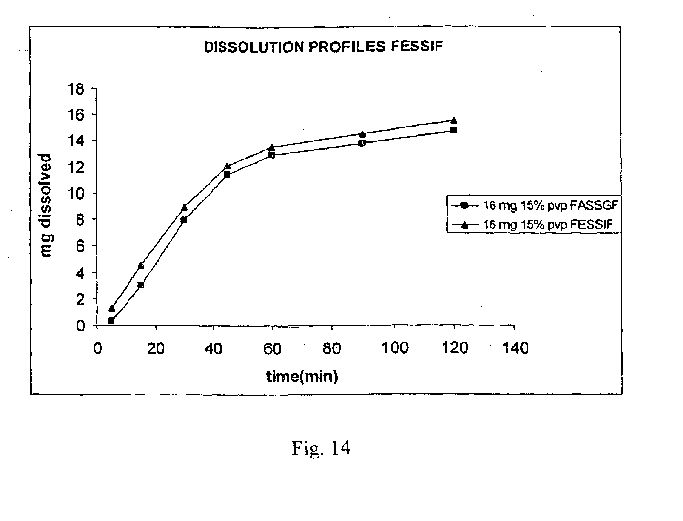

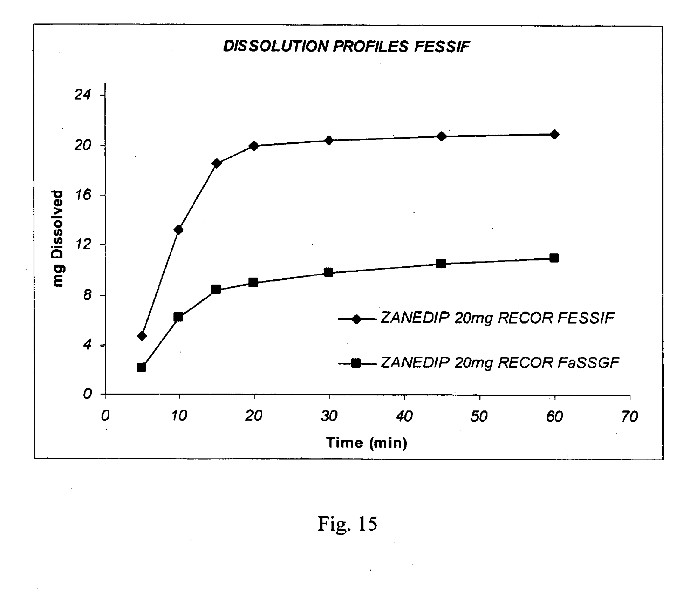

Based on such results it appears that, under these experimental conditions, the 16 mg formulation of example 4 has no food effect whereas that of the 8 mg formulation of example 5 has been significantly reduced. Such results are also supported by the in vitro data from simulated fed and fasting conditions, which shows approximatelly double solubilization (191%) of the drug under fed conditions when administered as Zanedip® 20 mg (see figure 15); on the other hand, the 16 mg formulation according to the present invention, tested under the same simulation methods, shows that approximately the same amount of drug is dissolved under fed and fasting conditions (see figure 14). The dissolution profiles obtained for both 16mg and 8 mg formulations provide an additional confirmation of the improved behaviour of the formulation of the present invention if compared to Zanedip® (see figures 10 and 11, respectively).

Independently on whether the same (20mg) or different dose (16/8mg) was administered to the volunteers, the compositions according to the present invention showed an enhanced bioavailability in comparison with the marketed reference product. This fact gives the possibility to manufacture a pharmaceutical composition with smaller quantity of active ingredient than the reference product but with the same effect resulting in better patient compliance and less side effects.

## Claims

1. A process for the preparation of a solid dosage form for oral administration such as a tablet, capsule or sachet containing Lercanidipine or a pharmaceutical acceptable salt thereof as an active ingredient and an effective amount of colloidal silicon dioxide, to enhance bioavailability, which comprises:
- dissolving the total quantity of said active ingredient, a portion of the total quantity of said colloidal silicon dioxide and a binder into water/EtOH solvent;
- adding to the formed solution the remaining portion of colloidal silicon dioxide and an optional excipient such as a diluent, a binder, a disintegrant, a glidant, a lubricant and wet granulating;
- dissolving a wetting agent such as Poloxamer into a small quantity of water/EtOH solvent and kneading with the first solution
- drying the wetted mass;
- sieving the dried mass and adding to the sieved mixture the total quantities of at least one optional excipient such as a binder, a diluent, a disintegrant, a lubricant and/or a glidant and mixing until uniform, and
- formulating the resulting mixture in a solid dosage form either by compressing it into a desired tablet form or by filling capsules or sachets.

2. A pharmaceutical composition for oral administration in solid dosage form obtainable from the process according to claim 1 comprising Lercanidipine or a pharmaceutical acceptable salt, thereof, as an active ingredient, and an amount of colloidal silicon dioxide, from 5% to 25% by weight, to enhance bioavailability and/or improve solubility.

3. The pharmaceutical composition according to claim 2, **characterized by** comprising from 7% to 20% by weight of colloidal silicon dioxide.

4. The pharmaceutical composition according to claim 2, **characterized by** comprising from 3% to 25% by weight of Lercanidipine or a pharmaceutical acceptable salt, thereof, preferably from 5% to 10% by weight, more preferably about 8% by weight.

5. The pharmaceutical composition according to claim 2, **characterized in that** the weight ratio of Lercanidipine or a pharmaceutical acceptable salt, thereof to colloidal silicon dioxide is from 10/1 to 1/60.

6. The pharmaceutical composition according to claim 2, **characterized by** comprising a binder, preferably polyvinylpyrrolidone (PVP), wherein said binder is present in amounts of from 5% to 20% by weight, preferably in amounts of up to about 15% by weight.

7. The pharmaceutical composition according to claim 2, **characterized by** comprising a disintegrant, preferably sodium starch glycolate, wherein said disintegrant is present in amounts of from 5% to 15% by weight, preferably of about 10% by weight.

8. The pharmaceutical composition according to claim 2, **characterized by** comprising a wetting agent, preferably a polyoxyethylene-polyoxypropylene copolymer (poloxamer), wherein said wetting agent is present in amounts of up to 5% by weight, preferably of about 2.5% by weight.

9. The pharmaceutical composition according to claim 2, **characterized by** comprising a diluent, preferably microcrystalline cellulose and/or lactose, more preferably in monohydrate form, wherein said diluent is present in amounts of from 40% to 65% by weight, preferably of from 45% to 60% by weight.

10. The pharmaceutical composition according to claim 2, **characterized by** comprising an internal phase and an external phase.

11. The pharmaceutical composition according to claim 10, **characterized in that** said external phase comprises, and preferably consists of, magnesium stearate.

12. The pharmaceutical composition according to claim 2, **characterized in that** Lercanidipine is present in hydrochloride form, preferably crystalline.

13. The pharmaceutical composition according to claim 2, **characterized by** containing Lercanidipine hydrochloride, lactose monohydrate, microcrystalline cellulose, sodium starch, glycolate, polyvinylpyrrolidone, colloidal silicon dioxide, polyoxyethylene-polyoxypropylene copolymer, magnesium stearate.

14. The pharmaceutical composition according to claim 2, **characterized in that** said solid dosage form is a tablet, capsule or sachet.

15. The pharmaceutical composition according to claim 14, **characterized in that** each solid dosage form contains from 7 to 9 mg or from 14 to 18 mg of Lercanidipine hydrochloride, preferably about 8 mg or about 16 mg of Lercanidipine hydrochloride.

16. The pharmaceutical composition according to claim 2, **characterized by** containing about 16 mg of Lercanidipine hydrochloride, about 80 mg of lactose monohydrate, about 16 mg of microcrystalline cellulose, about 20 mg of sodium starch glycolate, about 30 mg of polyvinylpyrrolidone, about 31 mg of colloidal silicon dioxide, about 5 mg of polyoxyethylene-polyoxypropylene copolymer, about 2 mg of magnesium stearate.

17. The pharmaceutical composition according to claim 2, **characterized by** containing about 8 mg of Lercanidipine hydrochloride, about 45 mg of lactose monohydrate, about 8 mg of microcrystalline cellulose, about 10 mg of sodium starch glycolate, about 10 mg of polyvinylpyrrolidone, about 15.50 mg of colloidal silicon dioxide, about 2.50 mg of polyoxyethylene-polyoxypropylene copolymer, about 1 mg of magnesium stearate.

## Patentansprüche

1. Verfahren zur Herstellung einer festen Dosis-Form zur oralen Verabreichung, wie als Tablette, Kapsel oder Päckchen, welche als aktiven Inhaltsstoff Lercanidipin oder ein pharmazeutisch annehmbares Salz davon enthält, und eine wirksame Menge kolloidales Siliziumdioxid, um die Bioverfügbarkeit zu steigern, welches umfasst:
- Auflösen der Gesamtmenge des aktiven Inhaltsstoffes, eines Teils der Gesamtmenge des kolloidalen Siliziumdioxids und eines Bindemittel in einem Wasser/EtOH-Lösungsmittel;
- Zusetzen des verbleibenden Teils kolloidalen Siliziumdioxids und eines gewählten Exzipienten, wie eines Lösungsmittels, eines Bindemittels, eines Zersetzungsmittels, eines Gleitmittels, eines Schmiermittels zu der gebildeten Lösung und Nassgranulieren,
- Auflösen eines Benetzungsmittels, wie Poloxamer in einer kleinen Menge Wasser/EtOH-Lösungsmittel und Kneten mit der ersten Lösung,
- Trocknen der benetzten Masse;
- Sieben der getrockneten Masse und Zusetzen der Gesamtmengen von mindestens einem optionalen Exzipienten, wie einem Bindemittel, einem Verdünnungsmittel, einem Zersetzungsmittel, einem Schmiermittel und/oder einem Gleitmittel zu dem gesiebten Gemisch, und Mischen bis einheitlich; und
- Formulierung des so erhaltenen Gemisches in einer festen Dosisform durch entweder Zusammenpressen in eine gewünschte Tablette oder durch Füllen von Kapseln oder Päckchen.

2. Pharmazeutische Zusammensetzung zur oralen Verabreichung in fester Dosisform, erhältlich gemäß dem Verfahren nach Anspruch 1, welche als aktiven Inhaltsstoff Lercanidipin oder ein pharmazeutisch annehmbares Salz davon enthält, und kolloidales Siliziumdioxid in einer Menge von 5 % bis 25 Gew.-%, um die Bioverfügbarkeit zu steigern oder die Löslichkeit zu verbessern.

3. Pharmazeutische Zusammensetzung nach Anspruch 2, **dadurch gekennzeichnet, dass** sie von 7% bis 20 Gew.-% kolloidales Siliziumdioxid enthält.

4. Pharmazeutische Zusammensetzung nach Anspruch 2, **dadurch gekennzeichnet, dass** sie von 3 % bis 25 Gew.-% Lercanidipin oder ein pharmazeutisch annehmbares Salz davon enthält, vorzugsweise von 5 % bis 10 Gew.-%, mehr bevorzugt etwa 8 Gew.-%.

5. Pharmazeutische Zusammensetzung nach Anspruch 2, **dadurch gekennzeichnet, dass** das Gewichtsverhältnis von Lercanidipin oder eines pharmazeutisch annehmbaren Salzes davon zu dem kolloidalen Siliziumdioxid von 10/1 bis 1/60 ist.

6. Pharmazeutische Zusammensetzung nach Anspruch 2, **dadurch gekennzeichnet, dass** sie ein Bindemittel, vorzugsweise Polyvinylpyrrolidon (PVP) umfasst, worin das Bindemittel in Mengen von 5 % bis 20 Gew.-%, vorzugsweise in Mengen von bis zu etwa 15 Gew.-% vorhanden ist.

7. Pharmazeutische Zusammensetzung nach Anspruch 2, **dadurch gekennzeichnet, dass** sie ein Zersetzungsmittel umfasst, vorzugsweise Natrium-Stärkeglycolat, worin das Zersetzungsmittel in Mengen von 5 % bis 15 Gew.-%, vorzugsweise etwa 10 Gew.-% vorhanden ist.

8. Pharmazeutische Zusammensetzung nach Anspruch 2, **dadurch gekennzeichnet, dass** sie ein Benetzungsmittel umfasst, vorzugsweise Polyoxyethylen-polyoxypropylen-Copolymer (Poloxamer), worin das Benetzungsmittel in Mengen von etwa 5 Gew.-%, vorzugsweise etwa 2,5 Gew.-% vorhanden ist.

9. Pharmazeutische Zusammensetzung nach Anspruch 2, **dadurch gekennzeichnet, dass** sie ein Verdünnungsmittel umfasst, vorzugsweise mikrokristalline Zellulose und/oder Lactose, mehr bevorzugt in Monohydrat-Form, worin das Verdünnungsmittel in Mengen von 40 % bis 65 Gew.-%, vorzugsweise von 45 % bis 60 Gew.-% vorhanden ist.

10. Pharmazeutische Zusammensetzung nach Anspruch 2, **dadurch gekennzeichnet, dass** sie eine innere Phase und eine äußere Phase umfasst.

11. Pharmazeutische Zusammensetzung nach Anspruch 10, **dadurch gekennzeichnet, dass** die externe Phase Magnesiumstearat umfasst, vorzugsweise daraus besteht.

12. Pharmazeutische Zusammensetzung nach Anspruch 2, **dadurch gekennzeichnet, dass** Lercanidipin in Hydrochlorid-Form vorhanden ist, vorzugsweise in kristalliner Form.

13. Pharmazeutische Zusammensetzung nach Anspruch 2, **dadurch gekennzeichnet, dass** sie Lercanidipin-Hydrochlorid, Lactose-Monohydrat, mikrokristalline Cellulose, Natrium-Stärkeglycolat, Polyvinylpyrrolidon, kolloidales Siliziumdioxid, Polyoxyethylen-polyoxypropylen-Copolymer, Magnesiumstearat enthält.

14. Pharmazeutische Zusammensetzung nach Anspruch 2, **dadurch gekennzeichnet, dass** die feste Dosisform eine Tablette, eine Kapsel oder ein Päckchen ist.

15. Pharmazeutische Zusammensetzung nach Anspruch 14, **dadurch gekennzeichnet, dass** jede feste Dosisform von 7 bis 9 mg oder von 14 bis 18 mg Lercanidipin-Hydrochlorid enthält, vorzugsweise etwa 8 mg oder etwa 16 mg Lercanidipin-Hydrochlorid.

16. Pharmazeutische Zusammensetzung nach Anspruch 14, **dadurch gekennzeichnet, dass** sie etwa 16 mg Lercanidipin-Hydrochlorid, etwa 80 mg Lactose-Monohydrat, etwa 16 mg mikrokristalline Cellulose, etwa 20 mg Natrium-Stärkeglycolat, etwa 30 mg Polyvinylpyrrolidon, etwa 31 mg kolloidales Siliziumdioxid, etwa 5 mg Polyoxyethylen-polyoxypropylen-Copolymer, etwa 2 mg Magnesiumstearat enthält.

17. Pharmazeutische Zusammensetzung nach Anspruch 14, **dadurch gekennzeichnet, dass** sie etwa 8 mg Lercanidipin-Hydrochlorid, etwa 45 mg Lactosemonohydrat, etwa 8 mg mikrokristalline Cellulose, etwa 10 mg Natrium-Stärkeglycolat, etwa 10 mg Polyvinylpyrrolidon, etwa 15,50 mg kolloidales Siliziumdioxid, etwa 2,50 mg Polyoxyethylen-polyoxypropylen-Copolymer, etwa 1 mg Magnesiumstearat enthält.

## Revendications

1. Procédé de préparation d'une forme galénique solide pour une administration par voie orale telle qu'un comprimé, une gélule ou un sachet contenant de la lercanidipine ou un sel pharmaceutiquement acceptable de celle-ci en tant que principe actif et une quantité efficace de dioxyde de silicium colloïdal, pour améliorer la biodisponibilité, qui consiste à :
- dissoudre la quantité totale dudit principe actif, une partie de la quantité totale dudit dioxyde de silicium colloïdal et un liant dans un solvant eau/EtOH ;
- ajouter à la solution formée la partie restante du dioxyde de silicium colloïdal et un excipient facultatif tel qu'un diluant, un liant, un délitant, un agent de glissement, un lubrifiant et un agent de granulation à l'état humide ;
- dissoudre un agent mouillant tel qu'un poloxamère dans une petite quantité du solvant eau/EtOH et malaxer avec la première solution ;
- sécher la masse mouillée ;
- tamiser la masse séchée et ajouter au mélange tamisé les quantités totales d'au moins un excipient facultatif tel qu'un liant, un diluant, un délitant, un lubrifiant et/ou un agent de glissement et mélanger jusqu'à uniformité ; et
- formuler le mélange résultant en une forme galénique solide en le comprimant en une forme de comprimé souhaitée ou en remplissant des gélules ou des sachets.

2. Composition pharmaceutique pour une administration par voie orale sous une forme galénique solide pouvant être obtenue par le procédé selon la revendication 1, comprenant de la lercanidipine ou un sel pharmaceutiquement acceptable de celle-ci, en tant que principe actif, et une quantité de dioxyde de silicium colloïdal, de 5 % à 25 % en poids, pour améliorer la biodisponibilité et/ou améliorer la solubilité.

3. Composition pharmaceutique selon la revendication 2, **caractérisée en ce qu'**elle comprend de 7 % à 20 % en poids de dioxyde de silicium colloïdal.

4. Composition pharmaceutique selon la revendication 2, **caractérisée en ce qu'**elle comprend de 3 % à 25 % en poids de lercanidipine ou d'un sel pharmaceutiquement acceptable de celle-ci, de préférence de 5 % à 10 % en poids, plus préférablement environ 8 % en poids.

5. Composition pharmaceutique selon la revendication 2, **caractérisée en ce que** le rapport pondéral de la lercanidipine ou d'un sel pharmaceutiquement acceptable de celle-ci au dioxyde de silicium colloïdal est de 10/1 à 1/60.

6. Composition pharmaceutique selon la revendication 2, **caractérisée en ce qu'**elle comprend un liant, de préférence de la polyvinylpyrrolidone (PVP), ledit liant étant présent en des quantités de 5 % à 20 % en poids, de préférence en des quantités allant jusqu'à environ 15 % en poids.

7. Composition pharmaceutique selon la revendication 2, **caractérisée en ce qu'**elle comprend un délitant, de préférence le glycolate d'amidon sodique, ledit délitant étant présent en des quantités de 5 % à 15 % en poids, de préférence en des quantités d'environ 10 % en poids.

8. Composition pharmaceutique selon la revendication 2, **caractérisée en ce qu'**elle comprend un agent mouillant, de préférence un copolymère de polyoxyéthylène-polyoxypropylène (poloxamère), ledit agent mouillant étant présent en des quantités allant jusqu'à 5 % en poids, de préférence d'environ 2,5 % en poids.

9. Composition pharmaceutique selon la revendication 2, **caractérisée en ce qu'**elle comprend un diluant, de préférence une cellulose microcristalline et/ou du lactose, plus préférablement sous une forme monohydrate, ledit diluant étant présent en des quantités de 40 % à 65 % en poids, de préférence de 45 à 60 % en poids.

10. Composition pharmaceutique selon la revendication 2, **caractérisée en ce qu'**elle comprend une phase interne et une phase externe.

11. Composition pharmaceutique selon la revendication 10, **caractérisée en ce que** ladite phase externe comprend du, et se compose de préférence de, stéarate de magnésium.

12. Composition pharmaceutique selon la revendication 2, **caractérisée en ce que** la lercanidipine est présente sous forme de chlorhydrate, de préférence sous forme cristalline.

13. Composition pharmaceutique selon la revendication 2, **caractérisée en ce qu'**elle contient du chlorhydrate de lercanidipine, du lactose monohydraté, de la cellulose microcristalline, du glycolate d'amidon sodique, de la polyvinylpyrrolidone, du dioxyde de silicium colloïdal, un copolymère de polyoxyéthylène-polyoxypropylène, du stéarate de magnésium.

14. Composition pharmaceutique selon la revendication 2, **caractérisée en ce que** ladite forme galénique solide est un comprimé, une gélule ou un sachet.

15. Composition pharmaceutique selon la revendication 14, **caractérisée en ce que** chaque forme galénique solide contient de 7 à 9 mg ou de 14 à 18 mg de chlorhydrate de lercanidipine, de préférence environ 8 mg ou environ 16 mg de chlorhydrate de lercanidipine.

16. Composition pharmaceutique selon la revendication 2, **caractérisée en ce qu'**elle contient environ 16 mg de chlorhydrate de lercanidipine, environ 80 mg de lactose monohydraté, environ 16 mg de cellulose cristalline, environ 20 mg de glycolate d'amidon sodique, environ 30 mg de polyvinylpyrrolidone, environ 31 mg de dioxyde de silicium colloïdal, environ 5 mg de copolymère de polyoxyéthylène-polyoxypropylène, environ 2 mg de stéarate de magnésium.

17. Composition pharmaceutique selon la revendication 2, **caractérisée en ce qu'**elle contient environ 8 mg de chlorhydrate de lercanidipine, environ 45 mg de lactose monohydraté, environ 8 mg de cellulose cristalline, environ 10 mg de glycolate d'amidon sodique, environ 10 mg de polyvinylpyrrolidone, environ 15,50 mg de dioxyde de silicium colloïdal, environ 2,50 mg de copolymère de polyoxyéthylène-polyoxypropylène, environ 1 mg de stéarate de magnésium.
